# EUROPEAN PATENT APPLICATION

(11) **EP 3 632 337 A1**
(43) Date of publication of application: **08.04.2020**
(21) Application number: 18810771.8
(22) Date of filing: 17.05.2018
(51) Int. Cl.: A61B 17/00

(54) **MEDICAL APPARATUS**

(30) Priority: 02.06.2017 CN 201710408126
(71) Applicant: Shanghai MicroPort Medical (Group) Co., Ltd., Shanghai 201203 (CN)
(72) Inventor: WANG, Zhen, Shanghai 201203 (CN); ZHOU, Yi, Shanghai 201203 (CN); HU, Yan, Shanghai 201203 (CN); YAO, Yao, Shanghai 201203 (CN); LI, Junfei, Shanghai 201203 (CN)
(74) Representative: Lavoix
(86) International application number: PCT/CN2018/087299
(87) International publication number: WO 2018/219151

(57) **Abstract**

A medical apparatus (10 and 20), which comprises a left atrial appendage occluder (1 and 4), a support frame transport element (31 and 61), a fixed part (2 and 5), and a fixed transport element (32 and 62). The support frame transport element (31 and 61) are provided with a first channel and a first connecting extremity provided at the distal extremity of the first channel. The left atrial appendage occluder (1 and 4) comprises a support frame body (11 and 41) and a support frame connecting element (12 and 42) provided on the support frame body (11 and 41). The support frame connecting element (12 and 42) is provided with a second channel and a second connecting extremity provided on the second channel. The first and second connecting extremities can be joined so as to allow the first and second channels to communicate and form a pushing channel. The distal extremity of the fixed transport element (32 and 62) can be joined with the fixed part (2 and 5) so as to allow the fixed transport element (32 and 62) to push in the pushing channel the fixed part (2 and 5) to enter a preset area, thus connecting the left atrial appendage occluder (1 and 4) to the preset area. The medical apparatus (10 and 20) facilitates the left atrial appendage occluder (1 and 4) to easily implement repeated positioning and retraction, neither damages a sheath nor experiences the problem of positioning failure caused by anchor detachment as a result of coming into contact with the sheath, thus improving the safeness of a surgery.

## Description

### TECHNICAL FIELD

The present application relates to the technical field of medical instruments, and in particular, to a medical device.

### BACKGROUND

Atrial Fibrillation (AF) is the common continuous arrhythmia in clinic. The incidence of AF in the general population is 0.5-1.3%. The main hazard of AF is to promote thrombosis. Complications such as stroke and peripheral vascular embolization caused by thrombus detachment significantly increase the disability and fatality rate. Stroke is the most common and devastating complication of AF. About 15 million people worldwide suffer from stroke each year, 20-25% of which are attributed to AF. The study suggests that 60% of patients with rheumatic heart disease have a cardiogenic thrombus from the left atrial appendage, and more than 90% of thrombi in the patients with non-valvular AF are formed in the left atrial appendage. Therefore, the intervention of the left atrial appendage to prevent thromboembolism in patients with AF, especially stroke, has important theoretical and clinical significance.

Anticoagulant therapy is a routine method for preventing complications of stroke and AF at present, but has certain limitations. Therefore, the use of safer and more effective measures to prevent AF and stroke is of great significance. At present, medical interventions are mostly used to occlude the left atrial appendage. The commonly used left atrial appendage occluder has a single plug type occluder represented by WATCHMAN and a plug-and-disc type left atrial appendage occluder represented by AMPLATZER Cardiac Plug (ACP).

The WATCHMAN occluder has a nickel-titanium alloy frame with a self-expanding structure and barbs fixed around. The surface of the occluder towards atrial is covered with a porous polytetrafluoroethylene membrane for blood inflow outflow in the left atrial appendage. The inventors have found that after the WATCHMAN occluder is plugged into the left atrial appendage, the entrance of left atrial appendage cannot be completely occluded since the shape of the entrance of the left atrial appendage is irregular, and the occluder has a limited deformability. Therefore, it is difficult to eliminate the thrombus caused by leakage of the left atrial appendage due to AF. In addition, the left atrial appendage has different structures, such as single-cavity structure and multi-cavity structure, and has different depths. The plug type occluder is unable to fully adapt to the anatomical structures of all left atrial appendages as well as to achieve a stable fixation. The ACP occluder is a two-disc type device consisting of a butterfly blade and a butterfly cap. The butterfly blade and the butterfly cap are connected by a concave waist. The butterfly blade is placed in the left atrial appendage to prevent the occluder from shifting. The butterfly cap seals the entrance of the left atrial appendage.

In addition, the plug-and-disc type left atrial appendage occluder has a structure in which the sealing disc and the fixed disc are integrated, and cannot be completely deformed independently. After the plug is plugged into the left atrial appendage, the disc portion latched at the entrance of the left atrial appendage is subjected to the pull of the plug portion, so that the disc portion cannot fully fit to the entrance of the left atrial appendage, and thus it is difficult to achieve an optimal occluding effect. Moreover, since the length adjustment of the plug portion and the disc portion is limited, it is difficult to achieve the optimal fixation and blood flow blockage, and the disc structure does not adapt to different lumen shapes of the left atrial appendage.

Both the plug type left atrial appendage occluder and the plug-and-disc type left atrial appendage occluder have the problems of incomplete sealing, easy peripheral leakage, inadaptability to the left atrial appendages with different shapes and depths, easy thrombosis formation, easy detachment and the like. Moreover, most of the existing left atrial appendage occluders are fixed by the anchor structure disposed thereon. There are two main types of anchor structures: one is that a plurality of filaments assembled on the occluder through suture lines, which is difficult to penetrate into the wall of the left atrial appendage and cannot be fixed; and the other is an anchor with sufficient puncture force, which cannot achieve repeated positioning and hinders the retrieval of the occluder as well as easily causes complications, and even has the risk of penetrating through the left atrial appendage.

### SUMMARY

It is an object of the present application to provide a medical device for solving one or more of the problems of difficult retrieval and positioning, difficult fixation, poor fixation effect and oversize of the delivery sheath that are presented in the existing left atrial appendage occluder.

To achieve the foregoing object and other related objects, the present application provides a medical device for placing a left atrial appendage occluder in the body, comprising the left atrial appendage occluder, a stent delivery member, a fixed portion, and a fixed delivery member, wherein the stent delivery member has a first channel and a first connecting end provided at a distal end of the first channel, and the left atrial appendage occluder comprises a stent body and a stent connector, the stent connector being disposed on the stent body, the stent connector having a second channel and a second connecting end provided at the second channel.

The second connecting end is able to engage with the first connecting end, so as to communicate the first connecting end with the second connecting end to form a push channel. A distal end of the fixed delivery member is able to engage with the fixed portion, so as to drive, inside the push channel, the fixed portion into a preset region by the fixed delivery member to connect the left atrial appendage occlude to the preset region.

Optionally, the fixed portion has a plurality of extensions. The plurality of extensions have proximal ends thereof connected to each other and are configured to engage with the distal end of the fixed delivery member and/or engage with the stent connector.

Optionally, the plurality of extensions are able to extend towards a plurality of directions after projecting out of the push channel under the drive of the fixed delivery member.

Optionally, the fixed portion comprises a fixed connector. The proximal ends of the plurality of extensions are connected to the fixed connector, and the fixed connector is configured to be connected to the stent connector and/or the fixed delivery member.

Optionally, at least some of the plurality of extensions pass through the stent body into the preset region.

Optionally, for each extension, the length is greater than the thickness and width.

Optionally, the fixed portion has a first dimension and a second dimension. The first dimension is smaller than the second dimension. The second dimension is configured to approach the inner surface of the stent body, and the first dimension is configured to approach the outer surface of the stent body.

Optionally, the fixed delivery member comprises a main body portion and a deflectable portion. An extension line of the deflectable portion is able to form an angle with an extension line of the main body portion.

Optionally, a distal end of the fixed portion is a helical structure or an anchor.

Optionally, the fixed portion is T-shaped.

In summary, the medical device provided in the present application has the following beneficial effects.

Firstly, the medical device releases the left atrial appendage occluder at the left atrial appendage through the stent delivery member, and then drives, inside the push channel, the fixed portion by the fixed delivery member to release and fix the left atrial appendage, so as to connect the left atrial appendage occluder to the left atrial appendage. In this way, it is unnecessary to provide an additional fixing mechanism such as an anchor on the left atrial appendage occluder. Therefore, the obstruction of the anchor can be eliminated during repeated positioning and retrieving, so that the left atrial appendage occluder can be smoothly sheathed to enable easy repeated positioning and retrieving. Moreover, after being sheathed, the left atrial appendage occluder does not damage the sheath canal, or result in the problem of positioning failure caused by detachment of anchor thorn due to contact with the sheath canal. Therefore, it is not easy to detach, and the reliability is high.

Secondly, the fixed portion of the medical device is able to adjust its location relative to the left atrial appendage occluder through the fixed delivery member, to penetrate into the left atrial appendage wall at different locations of the left atrial appendage occluder, so as to ensure as far as possible that each fixed portion can be penetrated into the left atrial appendage wall, which not only improves the utilization rate of the member, but also is more stable and not easy to fall off.

Thirdly, since the left atrial appendage occluder does not have an anchor, the structure of the left atrial appendage occluder is simplified, and the size of the sheath canal for delivering the left atrial appendage occluder is reduced, so that wounds, infection and complications caused by the sheath canal be accordingly reduced, which facilitates physical recovery of patients and improves the success rate of the operation.

### BRIEF DESCRIPTION OF THE DRAWINGS

FIG. 1 is a schematic diagram showing the positioning and fixation of a left atrial appendage occluder in a left atrial appendage when a delivering portion of a medical device according to Embodiment 1 of the present application is not withdrawn from a human body;
FIG. 2 is a schematic diagram showing the positioning and fixation of a left atrial appendage occluder in the left atrial appendage after the delivering portion of the medical device according to Embodiment 1 of the present application is withdrawn from the human body;
FIG. 3 is a schematic diagram showing the positioning and fixation of a hollow left atrial appendage occluder in a left atrial appendage according to Embodiment 1 of the present application;
FIG. 4 is a schematic diagram showing the positioning and fixation of a U-shaped left atrial appendage occluder in a left atrial appendage according to Embodiment 1 of the present application;
FIG. 5 is a schematic diagram showing the positioning and fixation of a cage-like left atrial appendage occluder formed by cutting in a left atrial appendage according to Embodiment 1 of the present application;
FIG. 6 is a schematic diagram showing the positioning and fixation of a funnel-shaped left atrial appendage occluder in a left atrial appendage according to Embodiment 1 of the present application;
FIG. 7 is a schematic diagram showing the positioning and fixation of an externally-occluding left atrial appendage occluder at an entrance of a left atrial appendage according to Embodiment 1 of the present application;
FIG. 8 is a schematic diagram showing the positioning and fixation of a left atrial appendage occluder in a left atrial appendage when a delivering portion of a medical device according to Embodiment 2 of the present application is not withdrawn from a human body;
FIG. 9 is a schematic diagram showing the positioning and fixation of the left atrial appendage occluder in the left atrial appendage after the delivering portion of the medical device according to Embodiment 2 of the present application is withdrawn from the human body;
FIG. 10 is a schematic diagram showing that a fixed portion of a medical device according to Embodiment 2 of the present application is driven by a motor to rotate into a left atrial appendage wall; and
FIG. 11 is a schematic diagram showing the positioning and fixation of a left atrial appendage occluder having a trumpet-shaped opening in a left atrial appendage according to Embodiment 2 of the present application.

### Description of reference numerals:

10, 20-medical device; 1, 4-left atrial appendage occluder; 2, 5-fixed portion; 3, 6-delivery portion; 11, 41-stent body; 12, 42-stent connector; 13, 43-occluding membrane; 21-extension; 22-fixed connector; 51-fixed structure; 31, 61-stent delivery member; 32, 62-fixed delivery member; 621-main body portion; 622-deflectable portion; 623-driving portion; S-left atrial appendage.

### DETAILED DESCRIPTION OF THE INVENTION

The core concept of the present application is to provide a medical device for placing a left atrial appendage occluder inside a body, comprising the left atrial appendage occluder, a stent delivery member, a fixed portion, and a fixed delivery member. The stent delivery member has a first channel and a first connecting end provided at a distal end of the first channel. The left atrial appendage occluder comprises a stent body and a stent connector. The stent connector is disposed on the stent body. The stent connector has a second channel and a second connecting end provided at the second channel. The second connecting end is engaged with the first connecting end, so as to communicate the first channel with the second channel to form a push channel. A distal end of the fixed delivery member is engaged with the fixed portion, so as to drive, inside the push channel, the fixed portion into a preset region by the fixed delivery member to connect the left atrial appendage occluder to the preset region. In the medical device, the left atrial appendage occluder and the fixed portion are able to be separately delivered and finally released independently, thereby allowing to reduce the size of the left atrial appendage occluder and solve the problems that the left atrial appendage occluder is difficult to retrieve smoothly and is not firmly secured.

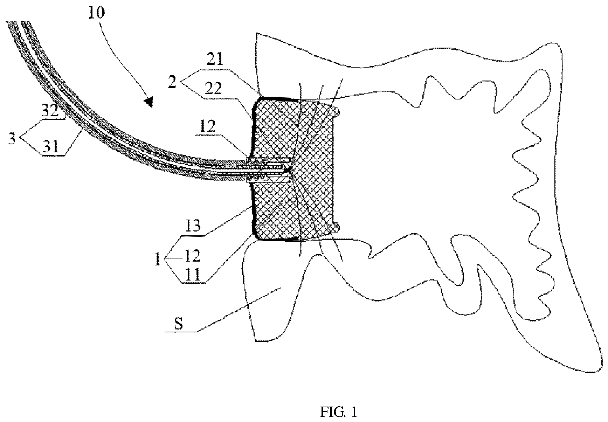

To make the objects, advantages and features of the present application clearer, the medical device proposed in the present application will be further described in detail below with reference to accompanying FIG. 1-FIG. 11 and specific embodiments. It should be noted that the drawings are in a very simplified form and not necessarily presented to scale, for the only purpose to facilitate convenient and explicit description of embodiments of the present application.

As used in the specification and the appended claims, the singular forms "a", "an" and "the" include plural objects, unless otherwise explicitly stated. As used in the specification and the appended claims, the term "or" is used in the meaning of "and/or", unless otherwise explicitly stated. The term "proximal end" generally refers to the end of a member close to the operator, and the term "distal end" refers to the end of the member away from the operator. As used in the specification and the appended claims, the terms "internal" and "inner surface" generally refer to the axial direction close to the member, and the terms "outer" and "outer surface" generally refer to the axial direction away from the member.

### Embodiment 1

FIG. 1 is a schematic diagram showing the positioning and fixation of a left atrial appendage occluder 1 in a left atrial appendage S when a delivering portion 3 of a medical device 10 according to Embodiment 1 of the present application is not withdrawn from a human body. As shown in FIG. 1, the medical device 10 includes a left atrial appendage occluder 1, a fixed portion 2, and a delivering portion 3. The left atrial appendage occluder 1 includes a stent body 11 and a stent connector 12. The fixed portion 2 includes an extension 21 and a fixed connector 22. The delivering portion 3 includes a stent delivery member 31 and a fixed delivery member 32.

The stent delivery member 31 has a first channel and a first connecting end located at a distal end of the first channel. The stent connector 12 is disposed on the stent body 11, and has a second channel and a second connecting end located on the second channel. The second connecting end matches with the first connecting end. Specifically, when the second connecting end is engaged with the first connecting end, the first channel is communicated with the second channel to form a delivery channel, and the stent delivery member 31 can push, inside the sheath canal, the left atrial appendage occluder 1 towards the distal end of the sheath canal, until the left atrial appendage occluder 1 is disengaged from the sheath canal and expands to release in the left atrial appendage S at a determined location.

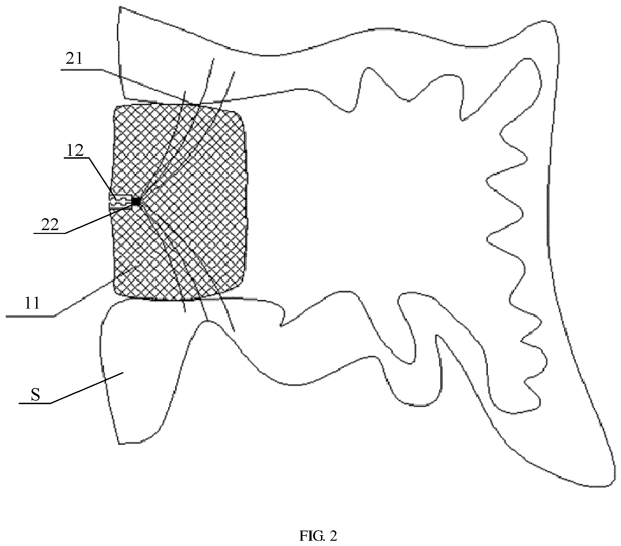

Next, a plurality of the extensions 21 are preferably provided, and the proximal ends of the plurality of extensions 21 are connected together through a fixed connector 22. In this way, after the left atrial appendage occluder 1 is positioned and released in the left atrial appendage S, the distal end of the fixed delivery member 32 is engaged with (detachably connected to) the fixed connector 22, so that the fixed delivery member 32 can push, inside the push channel, the fixed portion 2 towards the distal end of the push channel, until at least a part of each extension 21 protrudes out of the push channel and rebounds, which in turn makes at least a part of the extension 21 pass through the stent body 11 and penetrate into the left atrial appendage wall. After the positioning is confirmed to be correct, the sheath canal and the conveying portion 3 can be withdrawn from the human body as shown in FIG. 2 to complete the occlusion is completed. Obviously, the left atrial appendage wall here serves as the preset region provided in the present application. In addition, preferably, each of the plurality of extensions 21 passed through the stent body 11 and is fixed to the left atrial appendage wall so as to ensure that each extension 21 can be utilized to improve the utilization rate is improved and a firm fixation with an unlikely detachment can be achieved.

After the extension 21 is penetrated into the left atrial appendage wall, in order to connect the left atrial appendage occluder 1 to the left atrial appendage wall, the fixed portion 2 is also connected to the left atrial appendage occluder 1. In this embodiment, the fixed connector 22 of the fixed portion 2 is connected to the stent connector 12 of the left atrial appendage occluder 1, so that the left atrial appendage occluder 1 can be connected to the left atrial appendage wall. Alternatively, the extension 21 can form a connector thereon, and can be connected to the stent body 11 through the connector, where the connector can be a barb or the like.

Preferably, the fixed delivery member 32 can also release the connection between the fixed portion 2 and the left atrial appendage occluder 1 to facilitate repeated positioning and retrieving of the left atrial appendage occluder 1. Optionally, the fixed connector 22 is detachably connected to the stent connector 12 through a snap fastener, a thread, or the like, which is not limited specifically.

In combination with the beneficial effects, the left atrial appendage occluder 1 and the fixed portion 2 of the medical device 10 are separately disposed, and the stent delivery member 31 drives the left atrial appendage occluder 1 to release at the left atrial appendage. Then, the fixed portion 2 is driven, inside the push channel, to penetrate into the left atrial appendage wall, and connected to the left atrial appendage occluder 1 through the fixed delivery member 32, so as to achieve the fixed connection between the left atrial appendage occluder 1 and the left atrial appendage wall, thereby eliminating the fixing mechanism such as the anchor on the left atrial appendage occlude. Therefore, on one hand, the size of the sheath canal for assembling the left atrial appendage occluder is able to be reduced, thereby reducing the trauma, infection and complications caused by the overlarge sheath canal passing through the blood vessel and improving the success rate of the operation. On the other hand, the obstruction of the anchor on the left atrial appendage occluder is able to be eliminated in the repeated positioning and retrieving, so that the left atrial appendage occluder can be smoothly sheathed. Therefore, it is easy to achieve repeated positioning and retrieving without damaging the sheath canal, ensuring the service life of the device .In addition, there is no problem of the anchor detachment due to contact with the sheath canal, and therefore, a firm fixation can be achieved to guarantee the success of the operation.

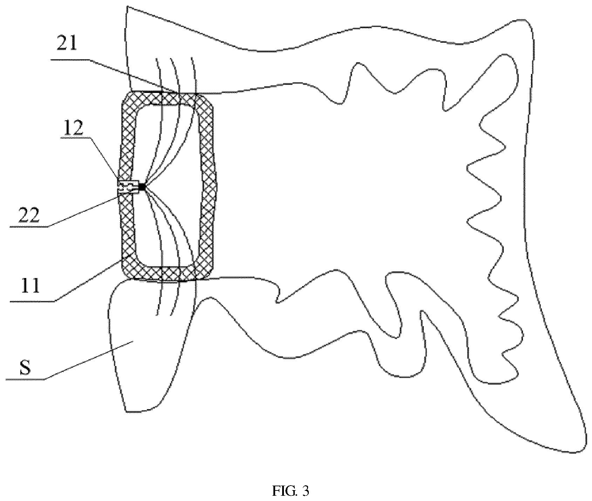

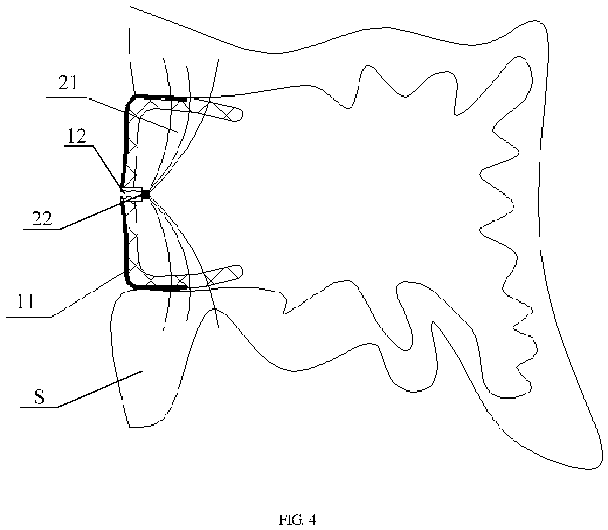

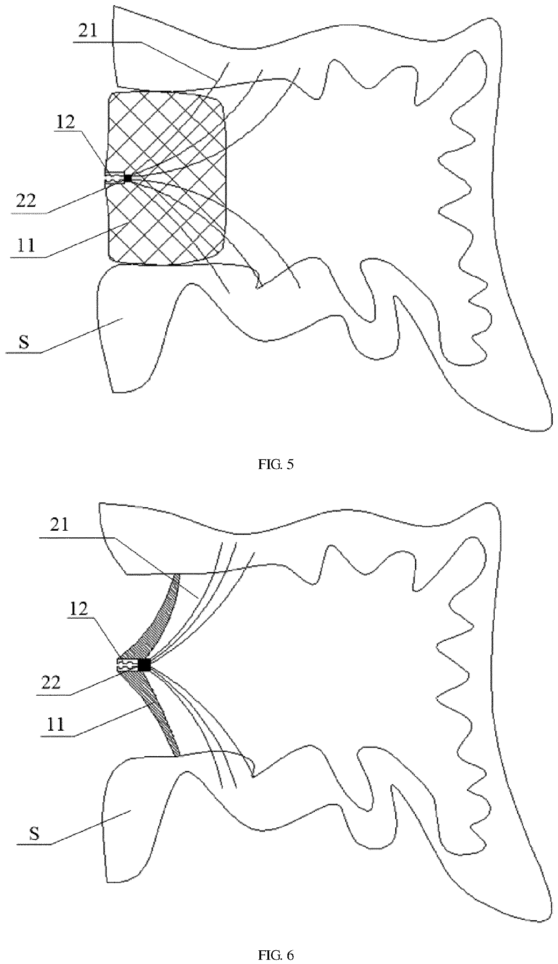

If the extension 21 of this embodiment penetrates into the left atrial appendage wall by passing through the stent body 11, the stent body 11 of this embodiment has the following structures:
As shown in FIG. 1, the stent body 11 is of a solid cage-like structure, which can be specifically manufactured by a weaving process. Alternatively, the stent body 11 can also be of a solid cage-like structure as shown in FIG. 2, in which an occlusion surface facing the interior of the left atrial appendage is not provided with a protrusion compared with FIG. 1. Certainly, the protrusion has no limitation to the stent body of the present application. Alternatively, as shown in FIG. 3, the stent body 11 is of a hollow cage-like structure, which is also manufactured by the weaving process. Alternatively, as shown in FIG. 4, the stent body 11 is of a U-shaped structure, which can be formed by a cutting process, and the opening of which faces the interior of the left atrial appendage. Alternatively, the solid cage-like structure shown in FIG. 2 can also be formed by the cutting process, as shown in FIG. 5.

However, the present application is not limited to the extension 21 penetrating into the left atrial appendage wall by passing through the stent body 11. As shown in FIG. 6, the plurality of extensions 21 penetrates into the left atrial appendage wall at one side of the stent body 11 facing the interior of the left atrial appendage. That is, the extension 21 directly penetrates into the left atrial appendage after projecting out of the push channel, and directly extends toward the direction of the left atrial appendage wall after rebounding. In this approach, the stent body 11 is funnel-shaped, with a funnel opening facing the interior of the left atrial appendage.

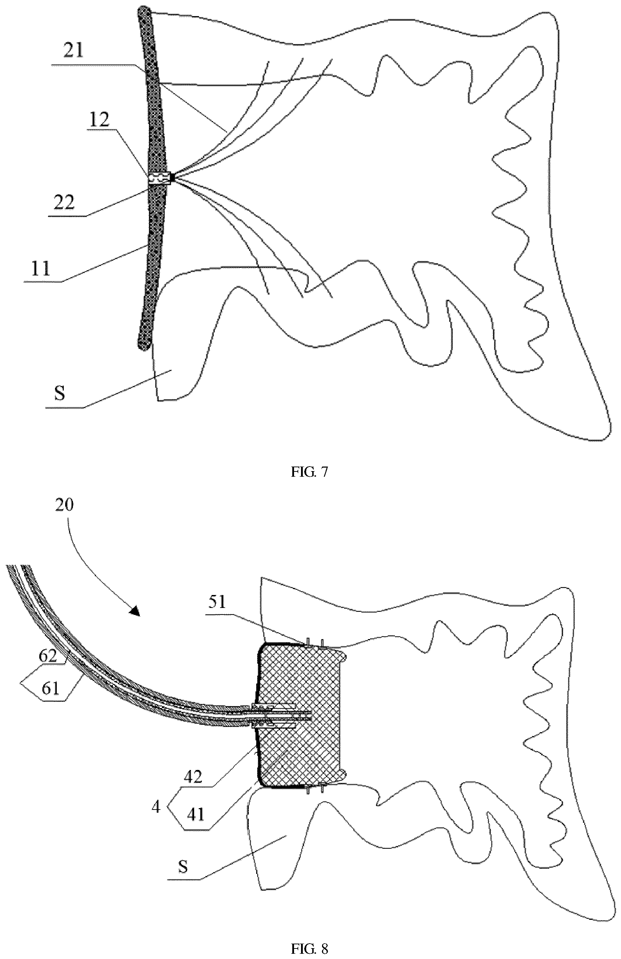

Obviously, provided above are internally-occluding left atrial appendage occluders. That is, the left atrial appendage occluder 1 is inserted inside the left atrial appendage, and an occlusion surface thereof is disposed facing the interior of the left atrial appendage. However, the left atrial appendage occluder of the present application can also be an externally-occluding structure, for example, as shown in FIG. 7. In an embodiment shown in FIG. 7, the left atrial appendage occluder 1 is not inserted inside the left atrial appendage, but is disposed at the entrance of the left atrial appendage with the occlusion surface facing the interior of the left atrial appendage. However, for the externally-occluding left atrial appendage occluder, the plurality of extensions 21 can also penetrate into the left atrial appendage wall without passing through the stent body 11. However, no matter for the internally-occluding or externally-occluding left atrial appendage occluder, the present application does not particularly limit whether the extensions 21 penetrate into the left atrial appendage wall by passing through the stent body 11, which can be specifically determined according to actual needs.

In this embodiment, the extension 21 specifically pass through meshes on the stent body 11, and different extensions 21 can pass through a same mesh or different meshes. The stent body 11 of this embodiment can be formed by weaving or cutting, and the shape thereof is not limited to an opening, cage-like, ellipsoidal, oval, funnel or trumpet, shape, etc. In addition, the mesh formed by cutting is not limited to the diamond-shaped or diamond-like mesh or the like. Preferably, the stent body 11 can be covered with a polymer membrane layer.

Further, after projecting out of the push channel under the driving of the fixed delivery member 32, and rebounding, the plurality of extensions 21 preferably extend towards a plurality of directions, so as to fix the left atrial appendage occluder from different locations and directions, thereby preventing the left atrial appendage from detaching. Optionally, the extension 21 can be made of an elongated rod or filament, or a plurality of intertwined filaments, as long as it can provide an axial force in the axial direction and can be smoothly penetrated into the left atrial appendage wall. In other words, the length of the extension 21 is much larger than the width and thickness of the extension 21 the extension 21. The length refers to an axial distance from the proximal end to the distal end of the extension 21. The width is the maximum size of the extension 21 in the first direction, and the thickness is the maximum size of the extension 21 in the second direction, where the first direction is perpendicular to the second direction, and the surface in which the first direction and the second direction are located is the cross section of the extension 21 (perpendicular to the axis). Preferably, the distal end of the extension 21 has a sharp tip, or is provided with a spiral structure or has a barb to facilitate smooth penetration into the left atrial appendage wall.

Moreover, the left atrial appendage occluder 1 also includes an occluding membrane 13 (specifically shown in FIG. 1 and FIG. 4). The occlusion surface or the non-occlusion surface is covered with the occluding membrane 13. Specifically, for the internally-occluding left atrial appendage occluder, the occluding membrane 13 is provided on the non-occlusion surface facing away from the interior of the left atrial appendage, and the externally-occluding left atrial appendage occluder is provided on the occlusion surface facing towards the interior of the left atrial appendage. The material of the occluding membrane 13 is preferably a polymer material, and is preferably a polymer material that is permeable to blood or impermeable to blood. The material of the stent body 11 can be a metal or a metal alloy (such as a magnesium alloy, a zinc alloy, a nickel-titanium alloy, and a cobalt-based alloy), and can also be a polymer material (such as polylactic acid (PLA)).

The stent connector 12 is disposed within the stent body 11 and is preferably disposed coaxial with the stent body 11 to effectively fix the left atrial appendage occluder. The stent body 11 and the stent connector 12 are of an integrated structure or a separated structure. For the externally-occluding left atrial appendage occluder, the distal end of the stent connector 12 preferably extends out of the occlusion surface to strengthen the connection between the stent connector 12 and the stent body 11. For the internally-occluding left atrial appendage occluder, the distal end of the stent connector 12 can either extend out of or not extend out of the occlusion surface. For example, in order to allow the extension 21 to penetrate into the left atrial appendage wall by passing through the stent body 11 from the interior of the stent body 11, the distal end of the stent connector 12 does not extend out of the occlusion surface. Otherwise, the distal end of the stent connector 12 extends out of the occlusion surface, so that the extension 21 penetrates into the left atrial appendage wall by passing through the stent body 11 from the exterior of the stent body 11 or directly penetrates into the left atrial appendage wall.

### Embodiment 2

The medical device 20 provided in this embodiment is basically the same as that provided in Embodiment 1, and the following description is only for different points.

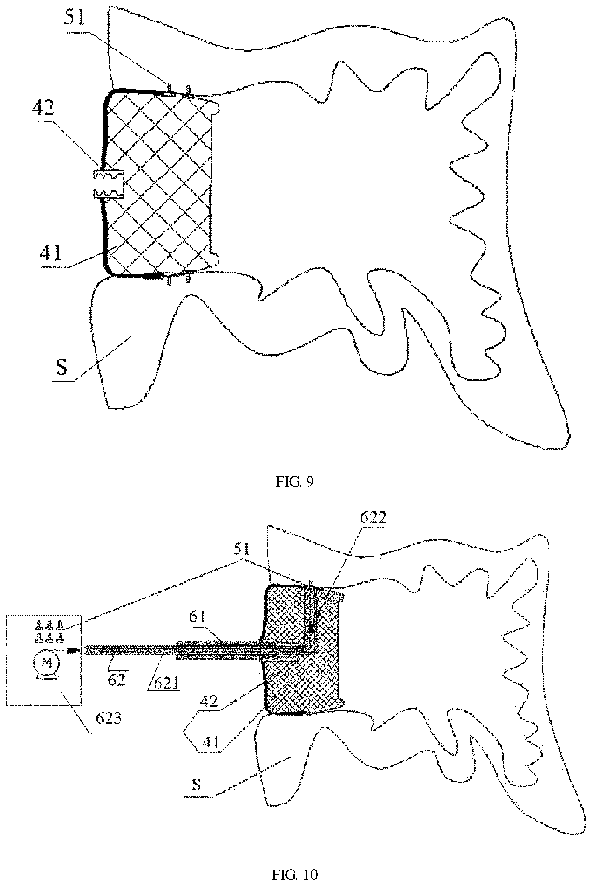

FIG. 8 is a schematic diagram showing the positioning and fixation of a left atrial appendage occluder 4 in a left atrial appendage when a delivering portion 6 of a medical device 20 according to Embodiment 2 of the present application is not withdrawn from a human body. FIG. 9 is a schematic diagram showing the positioning and fixation of the left atrial appendage occluder 4 in the left atrial appendage after the delivering portion 6 of the medical device 20 according to Embodiment 2 of the present application is withdrawn from the human body. FIG. 10 is a schematic diagram showing the delivery of the fixed portion 5 by the delivering portion 6 according to Embodiment 2 of the present application.

Referring to FIG. 8-FIG. 10, the medical device 20 includes a left atrial appendage occluder 4, a fixed portion 5, and a delivering portion 6. The left atrial appendage occluder 4 includes a stent body 41 and a stent connector 42. The fixed portion 5 includes a T-shaped fixed structure 51. The delivering portion 6 includes a stent delivery member61 and a fixed delivery member 62. The connection and working modes of the stent delivery member61 and the left atrial appendage occluder 4 in this Embodiment are the same as those of the stent delivery member61 and the left atrial appendage occluder 4 provided in Embodiment 1, and are not described in detail herein.

This embodiment differs from Embodiment 1 in that the fixed delivery member 62 includes a main body portion 621 and a deflectable portion 622 (specifically shown in FIG. 10) connected to each other. The deflectable portion 622 is axially bendable relative to the main body portion 621. That is, an extension line of the deflectable portion 622 forms an angle with an extension line of the main body portion 621, so that the fixed structure 51 is aimed at the left atrial appendage wall during delivery.

Specifically, after the left atrial appendage occluder 4 is released in the left atrial appendage S, the fixed structure 51 is engaged with the distal end of the deflectable portion 622. Then, the main body portion 621 drives the deflectable portion 622, inside the push channel, until the distal end of the deflectable portion 622 protrudes out of the push channel. In this way, the deflection angle of the deflectable portion 622 relative to the main body portion 621 is able to be adjusted to make the distal end of the deflectable portion 622 aim at the left atrial appendage wall in a predetermined direction. Next, the deflectable portion 622 is continuously driven to move, so as to make the fixed structure 51 at the distal end approach the left atrial appendage wall until a vertical portion of the fixed structure 51 passes through the stent body 41 and penetrates into the left atrial appendage wall, and then stops driving. Finally, the vertical portion of the fixed structure 51 penetrates into the left atrial appendage wall, and the horizontal portion of the fixed structure 41 hooks the stent body 41. In such a way, the left atrial appendage occluder 4 can be fixedly connected to the left atrial appendage wall, and the operation is convenient. In addition, if there is a plurality of fixed structures 51, the fixed structure 51 can penetrate into the left atrial appendage wall at different locations of the stent body 41 just by repeating the above process.

In this embodiment, the length of the deflectable portion 622 is not limited, and the deflectable portion 622 can be driven to bend by a deflection line connected to the deflectable portion 622. The location of the deflection line connected to the deflectable portion 622 is determined depending on the degree of bending. The length of the fixed delivery member 62 is at least greater than the length of the push channel, so that the deflectable portion 622 can extend out of the push channel to establish a delivery channel in the left atrial appendage that points to the left heart wall.

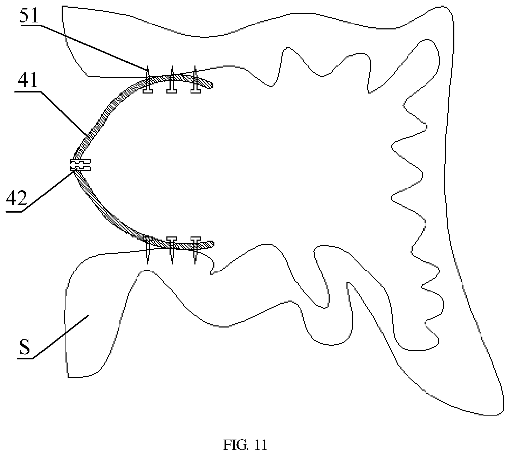

The present application is not limited to the establishment of a delivery channel in the stent body 41 that points to the left atrial appendage wall, which is identical to Embodiment 1. In an embodiment, as shown in FIG. 8 and FIG. 10, after extending out of the push channel, the deflectable portion 622 enters into the stent body 41 to establish a conveying channel inside the stent body 41. In another embodiment, as shown in FIG. 11, after extending out of the push channel, the deflectable portion 622enters into one side of the stent body 41 facing the interior of the left atrial appendage and establishes a delivery channel outside the stent body 41, so that the vertical portion of the fixed structure 51 penetrates into the left atrial appendage wall by passing through the stent body 41. In an embodiment shown in FIG. 11, the stent body 41 has a structure having a trumpet-shaped opening, and the deflectable portion 622 can be deflected in the trumpet-shaped opening after extending out of the delivery channel.

Next, if the fixed structure 51 is T-shaped, the vertical portion of the fixed structure 51 can be a spiral structure or a structure with a nose tool. However, the fixed structure 51 is not limited to the T-shape, as long as it has the first dimension and the second dimension. The first dimension is configured to approach the outer surface of the stent body 41, and the second dimension is configured to approach the inner surface of the stent body 41, the first dimension being smaller than the second dimension, such that a portion of the fixed structure 51 is located on one side of the stent body 11, and another portion of the fixed structure 51 is located on the other side of the stent body 41, the two sides being opposing, thereby anchoring the left atrial appendage occluder 4 to the left atrial appendage wall. It should be noted that the inner surface or one side of the stent body 41 herein refers to the axial direction close to the stent body, and the other side or the outer surface of the stent body refers to the axial direction away from the stent body.

In an embodiment, as shown in FIG. 10, the fixed delivery member 62 has a third channel. That is, the main body portion has an inner cavity, and the deflectable portion has another inner cavity communicated with the inner cavity. The two inner cavities form a third channel, and it is apparent that the third channel is bendable.

Preferably, the fixed delivery member 62 further includes a driving portion 623. In an embodiment, the driving portion 623 is configured to engage with the fixed structure 51 at the distal end of the third channel through the third channel, so as to drive the fixed structure 51 to penetrate into the left atrial appendage wall. Specifically, The use process is: after the fixed structure 51 is delivered to the predetermined location by the deflectable portion 622 (the predetermined location is sufficient for penetration of the fixed structure 51 into the left atrial appendage wall), the driving portion 623 is in contact with or connected to the horizontal portion of the fixed structure 51 at the distal end through the third channel, and in turn pushes the vertical portion of the fixed structure 51 to penetrate into the left atrial appendage wall. In the process of pushing the fixed structure 51 to penetrate into the left atrial appendage wall, the fixed structure 51 can be driven out of the deflectable portion 622 by the driving portion 623 and then rotatably screw into the left atrial appendage wall, or the fixed structure 51 can be directly pushed to move and penetrate into the left atrial appendage wall after the connection relationship between the fixed structure 51 and the deflectable portion 622 is released.

In another embodiment (not shown), the third channel only serves as a guide to create a deflectable channel in the body. Specifically, after the deflectable portion 622 is conveyed to the predetermined location, the fixed structure 51 is engaged with the distal end of the driving portion 623, and then the driving portion 623 guides the fixed structure 51 to the distal end of the deflectable portion 622 through the third channel until the vertical portion of the structure 51 penetrates into the left atrial appendage wall.

In this embodiment, the driving portion 623 includes a transmission mechanism and a motor. The motor is connected to the transmission mechanism. The transmission mechanism is configured to connect the fixed structure 51, and the motor drives the transmission mechanism to rotate or move. The transmission mechanism is for example a lead screw or other mechanism having a similar function.

In another preferred embodiment, the driving portion 623 can be a pneumatic tool that presses the fixed structure 51 into the left atrial appendage wall by compressed air.

Preferably, there are a plurality of fixed structures 51, and each fixed structure 51 can penetrated into the left atrial appendage wall through the modes provided in Embodiment 2. Therefore, the left atrial appendage occluder 4 can be fixed to the left atrial appendage at different locations.

Finally, in Embodiments 1 and 2, the medical device of the present application is described as a left atrial appendage occluder having a specific shape. However, the shape of the left atrial appendage occluder is not specifically limited in the present application, as long as occlusion can be realized.

In summary, the medical device provided in the present application has the following beneficial effects.

Firstly, the medical device releases the left atrial appendage occluder at the left atrial appendage through the stent delivery member, and then drives, inside the push channel, the fixed portion by the fixed delivery member to release and fix the left atrial appendage, so as to connect the left atrial appendage occluder to the left atrial appendage. In this way, it is unnecessary to provide an additional fixing mechanism such as an anchor on the left atrial appendage occluder. Therefore, the obstruction of the anchor can be eliminated during repeated positioning and retrieving, so that the left atrial appendage occluder can be smoothly sheathed to enable easy repeated positioning and retrieving. Moreover, after being sheathed, the left atrial appendage occluder does not damage the sheath canal, or result in the problem of positioning failure caused by detachment of anchor thorn due to contact with the sheath canal. Therefore, it is not easy to detach, and the reliability is high.

Secondly, the fixed portion of the medical device is able to adjust its location relative to the left atrial appendage occluder through the fixed delivery member, to penetrate into the left atrial appendage wall at different locations of the left atrial appendage occluder, so as to ensure as far as possible that each fixed portion can be penetrated into the left atrial appendage wall, which not only improves the utilization rate of the member, but also is more stable and not easy to fall off.

Thirdly, since the left atrial appendage occluder does not have an anchor, the structure of the left atrial appendage occluder is simplified, and the size of the sheath for delivering the left atrial appendage occluder is reduced, so that wounds, infection and complications caused by the sheath canal be accordingly reduced, which facilitates physical recovery of patients and improves the success rate of the operation.

The above description is only for the description of preferred embodiments of the present application, and is not intended to limit the scope of the present application. Any changes and modifications made by those skilled in the art according to the above disclosure are all within the protection scope of the appended claims.

## Claims

1. A medical device, for placing a left atrial appendage occluder inside a body, **characterized in** comprising the left atrial appendage occluder, a stent delivery member, a fixed portion, and a fixed delivery member, wherein the stent delivery member has a first channel and a first connecting end provided at a distal end of the first channel, and the left atrial appendage occluder comprises a stent body and a stent connector, the stent connector being disposed on the stent body, the stent connector having a second channel and a second connecting end provided at the second channel;
wherein the second connecting end is able to engage with the first connecting end, so as to connect the first connecting end to the second connecting end to form a push channel; a distal end of the fixed delivery member is able to engage with the fixed portion, so as to drive, inside the push channel, the fixed portion into a preset region by the fixed delivery member to connect the left atrial appendage occluder to the preset region.

2. The medical device according to claim 1, wherein the fixed portion has a plurality of extensions, and the plurality of extensions have proximal ends thereof connected to each other and are configured to engage with the distal end of the fixed delivery member and/or engage with the stent connector.

3. The medical device according to claim 2, wherein the plurality of extensions are able to extend towards a plurality of directions after projecting out of the push channel under the drive of the fixed delivery member.

4. The medical device according to claim 2, wherein the fixed portion comprises a fixed connector, and the proximal ends of the plurality of extensions are connected to the fixed connector, and wherein the fixed connector is configured to be connected to the stent connector and/or the fixed delivery member.

5. The medical device according to claim 2, wherein at least some of the plurality of extensions pass through the stent body into the preset region.

6. The medical device according to any one of claims 2 to 5, wherein a length of each extension is greater than a thickness and width thereof.

7. The medical device according to claim 1, wherein the fixed portion has a first dimension and a second dimension, and the first dimension is smaller than the second dimension, wherein the second dimension is configured to approach an inner surface of the stent body, and the first dimension is configured to approach an outer surface of the stent body.

8. The medical device according to claim 1, wherein the fixed delivery member comprises a main body portion and a deflectable portion, and an extension line of the deflectable portion is able to form an angle with an extension line of the main body portion.

9. The medical device according to claim 7, wherein a distal end of the fixed portion is a helical structure or an anchor.

10. The left atrial appendage occluder according to any one of claims 7 to 9, wherein the fixed portion is T-shaped.
